(19) **Europäisches Patentamt / European Patent Office / Office européen des brevets**

(11) **EP 2 512 325 B1**

(12) **EUROPEAN PATENT SPECIFICATION**

(45) Date of publication and mention of the grant of the patent:
**31.08.2016 Bulletin 2016/35**

(21) Application number: **10767734.6**

(22) Date of filing: **21.04.2010**

(51) Int Cl.:
**A61B 5/00** $^{(2006.01)}$

(86) International application number:
**PCT/US2010/031960**

(87) International publication number:
**WO 2010/124034 (28.10.2010 Gazette 2010/43)**

(54) **PROCESSING PHYSIOLOGICAL SENSOR DATA USING A PHYSIOLOGICAL MODEL COMBINED WITH A PROBABILISTIC PROCESSOR**

VERARBEITUNG VON PHYSIOLOGISCHEN SENSORDATEN MIT EINEM PHYSIOLOGISCHEN MODELL IN KOMBINATION MIT EINEM PROBABILISTISCHEN PROZESSOR

TRAITEMENT DE DONNÉES DE CAPTEUR PHYSIOLOGIQUE AU MOYEN D'UN MODÈLE PHYSIOLOGIQUE COMBINÉ À UN PROCESSEUR PROBABILISTIQUE

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **17.12.2009 US 640278**

(43) Date of publication of application:
**24.10.2012 Bulletin 2012/43**

(73) Proprietor: **Streamline Automation, LLC Hunstville, AL 35805 (US)**

(72) Inventor: **TEIXEIRA, Rodrigo, E. Madison AI 35758 (US)**

(74) Representative: **Zacco Sweden AB P.O. Box 5581 114 85 Stockholm (SE)**

(56) References cited:
**US-A1- 2003 018 457    US-A1- 2003 233 197**
**US-A1- 2006 190 217    US-A1- 2007 010 723**
**US-A1- 2008 183 090    US-A1- 2009 069 647**

- **SAATCI ET AL.: 'Dual Unscented Kalman Filter and Its Applications to Respiratory System Modeling', [Online] April 2009, pages 206 - 215, XP008153680 Retrieved from the Internet: <URL:http://www.intechopen.com/source/pdfs/ 6331/InTech-Dual_unscented_kalman_filter_and_its_applications_to_respiratory_system_modelling.pdf> [retrieved on 2009-11-11]**
- **GAHAHRAMANI: 'Unsupervised Learning', [Online] 16 September 2004, pages 1 - 10, XP008153681 Retrieved from the Internet: <URL:http://mlg.eng.cam.ac.uk/zoubin/papers /ul.pdf> [retrieved on 2011-11-11]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

BACKGROUND

Field of the Invention

The present invention relates generally to apparatus and methods for processing physiological sensor data and specifically to a pulse oximeter comprising a data processing system. The data processing system improves the accuracy of blood oxygen saturation and heart rate measurements made by the pulse oximeter and can be used to estimate stoke volume, cardiac output, and other cardiovascular and respiratory parameters.

Description of Related Art

Biomedical monitoring devices such as pulse oximeters, glucose sensors, electrocardiograms, capnometers, fetal monitors, electromyograms, electroencephalograms, and ultrasounds are sensitive to noise and artifacts. Typical sources of noise and artifacts include baseline wander, electrode-motion artifacts, physiological artifacts, high-frequency noise, and external interference. Some artifacts can resemble real processes, such as ectopic beats, and cannot be removed reliably by simple filters.

The influence of multiple sources of contaminating signals often overlaps the frequency of the signal of interest, making it difficult, if not impossible, to apply conventional filtering. Severe .artifacts such as occasional signal dropouts due to sensor movement or large periodic artifacts are also difficult to filter in real time. Biological sensor hardware can be equipped with a computer comprising software for post-processing data and reducing or rejecting noise and artifacts. Current filtering techniques typically use some knowledge of the expected frequencies of interest where the sought-after physiological information should be found, and do not contain a mathematical model describing either the physiological processes that are measured or the physical processes that measure the signal.

Adaptive filtering has been used to attenuate artifacts in pulse oximeter signals corrupted with overlapping frequency noise bands by estimating the magnitude of noise caused by patient motion and other artifacts, and canceling its contribution from pulse oximeter signals during patient movement. Such a time correlation method relies on a series of assumptions and approximations to the expected signal, noise, and artifact spectra, which compromises accuracy, reliability and general applicability.

Biomedical filtering techniques based on Kalman and extended Kalman techniques offer advantages over conventional methods and work well for filtering linear systems or systems with small nonlinearities and Gaussian noise. These filters, however, are not adequate for filtering highly nonlinear systems and non-Gaussian/non-stationary noise. Therefore, obtaining reliable biomedical signals continue to present problems, particularly when measurements are made in mobile, ambulatory and physically active patients.

Existing data processing techniques, including adaptive noise cancellation filters are unable to extract information that is hidden or embedded in biomedical signals and may also discard some potentially valuable information.

US 2009/0069647 discloses a method and system for monitoring arterial blood pressure, pulse oximetry, and intracranial pressure and calculating heart rate, respiratory rate, pulse pressure variation, harmonic phases, pulse morphology, using a statistical state-space model of cardiovascular signals and a generalized Kalman filter (EKF). The method is not able to extract information such as stroke volume and total blood volume that may be hidden or embedded in monitoring signals.

BRIEF SUMMARY OF THE INVENTION

The present invention fills a need in the art for biomedical monitoring devices capable of accurately and reliably measuring physiological parameters made in mobile, ambulatory and physically active patients. The present invention also provides for the processing of data measured by a biomedical monitoring device to extract additional information from a biomedical sensor signal to measure additional physiological paramaters. For instance, pulse oximeters are currently used to measure blood oxygen saturation and heart rate. A pulse oximetry signal, however, carries additional information that is extract using the present invention to estimate additional physiological parameters including left-ventricular stroke volume, aortic blood pressure, and systemic blood pressure.

One embodiment described herein is a pulse oximeter system comprising a data processor configured to perform a method that combines a sigma point Kalman filter (SPKF) or sequential Monte Carlo (SMC) algorithm with Bayesian statistics and a mathematical model comprising a cardiovascular model and a plethysmography model to remove contaminating noise and artifacts from the pulse oximeter sensor output to measure blood oxygen saturation, heart rate, left-ventricular stroke volume, aortic blood pressure, systemic blood pressure, and total blood volume.

Another embodiment is an electrocardiograph comprising a data processor performing a method combining a SPKF or SMC algorithm with Bayesian statistics and a mathematical model comprising a cardiovascular model including heart electrodynamics, electronic/contractile wave propagation and a model to remove contaminating noise and artifacts from electrode leads and sensor output to produce electrocardiograms.

The computational model includes variable state parameter output data that corresponds to a physiological parameter being measured to mathematically represent a current physiological state for a subject. The physiological parameter being measured is, most preferably, directly represented by a variable state parameter such that the value for the state parameter at time t is equal to an estimated value for the physiological parameter at time t. The estimated value of the physiological parameter being measured (estimated) may also correspond directly to (*i.e.* be equal to) the value of the model parameter at time t or the estimated value for the physiological parameter may be calculated from a state parameter, a model parameter, or a combination of one or more state and/or model parameters.

SPKF or SMC is used to generate a reference signal in the form of a first probability distribution from the model's current (time = *t*) physiological state. The reference signal probability distribution and a probability distribution generated from a measured signal from a sensor at a subsequent time (time = *t+n)* are convoluted using Bayesian statistics to estimate the true value of the measured physiological parameter at time = *t+n.* The probability distribution function may be discrete or continuous, and may identify the probability of each value of an unidentified random variable (discrete), or the probability of the value falling within a particular interval (continuous).

BRIEF DESCRIPTION OF THE DRAWINGS:

FIG. 1 is a flow chart showing the path of information flow from a biomedical sensor to a data a processor and on to an output display according to one embodiment of the invention.

FIG. 2 is a flow chart showing inputs, outputs, and conceptual division of model parts for a dynamic state-space model (DSSM).

FIG. 3 is a block diagram showing mathematical representations of inputs, output and conceptual divisions of the DSSM shown in FIG. 2.

FIG. 4 is a mathematical representation of the process of dual estimation.

FIG. 5 is a schematic diagram showing the process steps involved in a dual estimation process.

FIG. 6 is a mathematical representation of the process of joint estimation.

FIG. 7 is a schematic diagram showing the process steps involved in a joint estimation process.

FIG. 8 is a flow chart showing the components of a DSSM used for pulse oximetry data processing.

FIG. 9 is a flow chart showing examples of parameter inputs and outputs for a DSSM used for pulse oximetry data processing.

FIG. 10 is a flow chart showing the components of a DSSM used for electrocardiography data processing.

FIG. 11 is a chart showing input sensor data and processed output data from a data processor configured to process pulse oximetry data.

FIG. 12 is a chart showing input sensor data and processed output data from a data processor configured to process pulse oximetry data under a low blood perfusion condition.

FIG. 13 is a chart showing noisy non-stationary ECG sensor data input and processed heart rate and ECG output for a data processor configured to process ECG sensor data.

FIG. 14 is a chart showing input ECG sensor data and comparing output data from a data processor according to the present invention with output data generating using a Savitzky-Golay FIR data processing algorithm.

DETAILED DESCRIPTION OF THE INVENTION

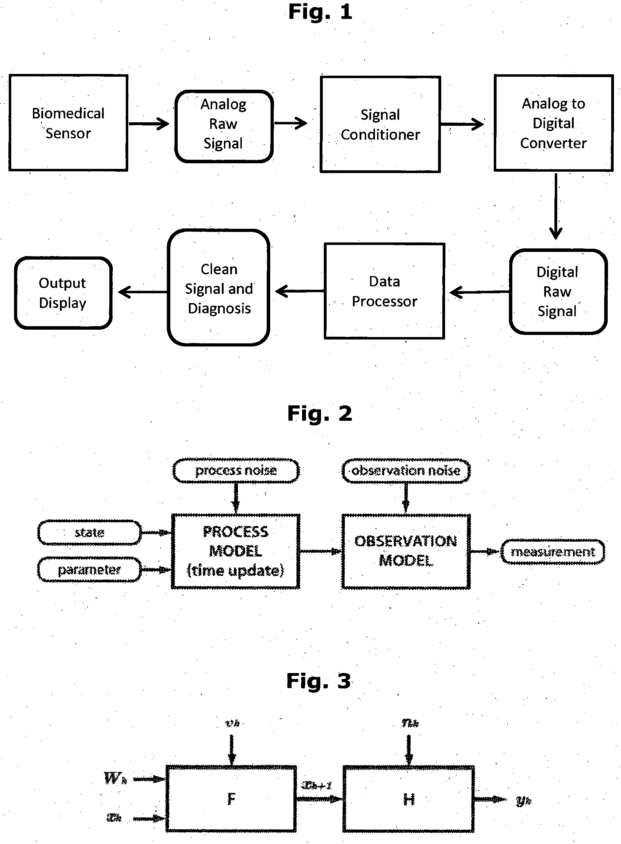

FIG. 1 shows an example of a top-level schematic for data processing according to the present invention. A biomedical sensor, usually associated with a biomedical monitoring device, normally produces a raw analog output signal that is converted to a raw digital output signal. The analog to digital conversion may also be accompanied by signal filtering or conditioning: Digital signals are received by a data processor configured to process the digital data and produce a processed (or clean) signal comprising an estimated true value for the physiological parameter being measured. The processed signal is then displayed, for example, in the form of an electronic, hard copy, audible, visual, and/or tactile output. The output may be used, for example, by a user to monitor a patient, by a user for self monitoring, or by a user as biofeedback process.

The data processor shown in FIG. 1 is configured to receive, as input data, digital signals from one or more biomedical sensors, and enter the data into a dynamic state-space model (DSSM) integrated with a processor engine. The integrated DSSM/Processor engine produces transformed output data that corresponds to a physiological parameter measured by the biomedical sensor(s), in the form of an estimated true value for the physiological parameter. The processor engine may operate in a dual estimation mode (a dual estimation engine) or in a joint estimation mode (a join estimation engine). The output may include additional outputs corresponding to physiological parameters not measured by the physiological sensor(s), diagnostic information, and a confidence interval representing the probability that the output estimated value(s) for the physiological parameter(s) is accurate. The data transformation process performed by the data processor may be used to remove artifacts from the input data to produce output data having higher accuracy than the input data and/or to extract information from the input data to generate output data estimating the values for physiological parameters that are not otherwise measured or reported using data from the sensor(s).

Mathematical and Computational Models

A mathematical model or a computer model, as used herein, involves the use of dependent state parameters and independent, variable, or constant model parameters in a mathematical representation of physiological processes that give rise to a physiological parameter being measured and processes through which sensor data is detected.

A mathematical model may include model and/or state parameters that correspond directly to physiological parameters including vital signs such as oxygen saturation of blood ($SpO_2$), heart rate (HR), respiratory rate (RR), and blood pressure (BP) that can be directly measured; physiological parameters not directly measured such as total blood volume (TBV), left-ventricular stroke volume (SV), vasomotor tone (VT), autonomous nervous system (ANS) tone, and stroke volume (SV); and hemoglobin-bound complexes, concentrations of metabolic intermediates, and concentrations of drugs present in one or more tissues or organs.

A mathematical model may also use mathematical representations of physiological observations that do not correspond directly to any physiological process, such as mathematical representations of signals obtained from sensor data or empirically fitting a mathematical equation to data collected from a physiological source.

While the scope of a mathematical model used in the context of the present invention cannot possibly encompass every single process of human physiology, it should have the capacity to interpret the measured observable(s). For instance, if the intent is to process electrocardiography (ECG) signals, a model describing the generation and propagation of electrical impulses in the heart should be included.

The fusion of two or more biomedical signals follows the same principle. For instance, if the intent is to measure blood pressure waves and electrocardiogram signals simultaneously, the use of a heart model describing both the electrical and mechanical aspects of the organ should be used. Initially, the model may also accept manual data input as a complement to data from sensors. Nonlimiting examples of manually entered data include food consumption over time vital signs, gender, age, weight, and height.

Non-physiological models may be included in and/or coupled to the DSSM in cases where non-biomedical signals are measured. For instance, one may use non-biomedical measurements to enhance or complement biomedical measurements. A non-limiting example is the use of accelerometer data to enhance motion artifact rejection in biomedical measurements. In order to accomplish this, the physiological model is extended to describe both measurements, which may include, in this example, cardiovascular circulation at rest, at different body postures (standing, supine, etc), and in motion.

Dynamic State-Space Model

**FIG. 2 and FIG. 3** show schematics of a dynamic state-space model (DSSM) used in the processing of data according to the present invention. The DSSM comprises a process model **F** that mathematically represents physiological processes involved in generating one or more physiological parameters measured by a biomedical sensor and describes the state of the subject over time in terms of state parameters. This mathematical model optimally includes mathematical representations accounting for process noise such as physiologically caused artifacts that may cause the sensor to produce a digital output that does not produce an accurate measurement for the physiological parameter being sensed. The DSSM also comprises an observational model **H** that mathematically represents processes involved in collecting sensor data measured by the biomedical sensor. This mathematical model optimally includes mathematical representations accounting for observation noise produced by the sensor apparatus that may cause the sensor to produce a digital output that does not produce an accurate measurement for a physiological parameter being sensed. Noise terms in the mathematical models are not required to be additive.

While the process and observational mathematical models may be conceptualized as separate models, they are normally integrated into a single mathematical model that describes processes that produce a physiological parameter and processes involved in sensing the physiological parameter. That model, in turn, is integrated with a processing engine within an executable program stored in a data processor that is configured to receive digital data from one or more sensors and to output data to a display or other output formats.

**FIG. 3** provides mathematical descriptions of the inputs and outputs corresponding to **FIG. 2.** Initially, values for state parameters, preferably in the form of a state vector $\mathbf{x}_k$, are received by the DSSM together with input model

parameters $W_k$. Process noise $\mathbf{v}_k$ and observation noise $n_k$ are also received by the DSSM, which updates the state parameter vector and model parameter vector and produces an output observation vector $\mathbf{y}_k$. Once the model is initialized, the updated state vector $\mathbf{x}_{k+1}$, updated model parameters $\mathbf{W}_{k+!}$, and time-specific sensor data are used as input for each calculation for subsequent iterations, or time steps.

The DSSM is integrated in a dual estimation processing engine or a joint estimation processing engine. The most favored embodiment makes use of a DSSM built into a Sigma point Kalman filter (SPKF) or Sequential Monte Carlo (SMC) processing engine. Sigma point Kalman filter (SPKF), as used herein, refers to the collective name used for derivativeless Kalman filters that employ the deterministic sampling based sigma point approach to calculate approximations of the optimal terms of the Gaussian approximate linear Bayesian update rule, including unscented, central difference, square-root unscented, and square-root central difference Kalman filters.

SMC and SPKF processing engines operate on a general nonlinear DSSM having the form:

$$\mathbf{x}_k = \mathbf{f}\left(\mathbf{x}_{k-1}, \mathbf{v}_{k-1}; \mathbf{W}\right) \qquad (1)$$

$$\mathbf{y}_k = \mathbf{h}(\mathbf{x}_k, \mathbf{n}_k; \mathbf{W}) \qquad (2)$$

A hidden system state, $\mathbf{x_k}$, propagates over time index, **k,** according to the system model, **f.** The process noise is $\mathbf{v_{k-1}}$, and **W** is the vector of model parameters. Observations, $\mathbf{y_k}$, about the hidden state are given by the observation model **h** and $\mathbf{n_k}$ is the measurement noise. When **W** is fixed, only state estimation is required and either SMC or SPKF can be used to estimate the hidden states.

Unsupervised Machine Learning

Unsupervised machine learning, sometimes referred to as system identification or parameter estimation, involves determining the nonlinear mapping:

$$\mathbf{y}_k = \mathbf{g}(x_k; w_k) \qquad (3)$$

where xk is the input, yk is the output, and the nonlinear map g(.) is parameterized by the model parameter vector **W.** The nonlinear map, for example, may be a feed-forward neural network, recurrent neural network, expectation maximization algorithm, or enhanced Kalman filter algorithm. Learning corresponds to estimating **W** in some optimal fashion. In the preferred embodiment, SPKF or SMC is used for updating parameter estimates. One way to accomplish this is to write a new state-space representation

$$w_{k+1} = w_k + r_k \qquad (4)$$

$$d_k = g(x_k; w_k) + e_k \qquad (5)$$

where $w_k$ correspond to a stationary process with identity state transition matrix, driven by process noise $r_k$. The desired output $d_k$ corresponds to a nonlinear observation on $w_k$.

Dual Estimation Engine for Estimation of State and Model Parameters

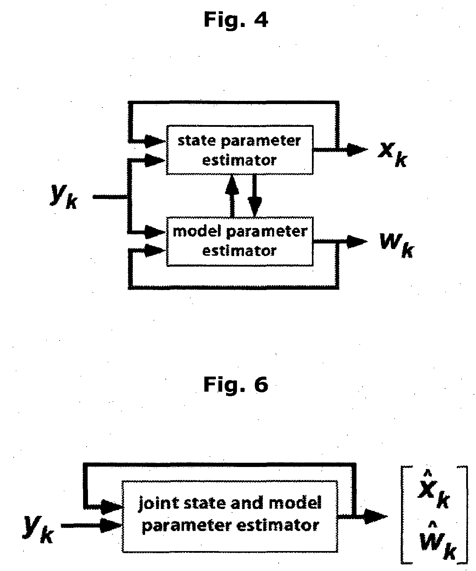

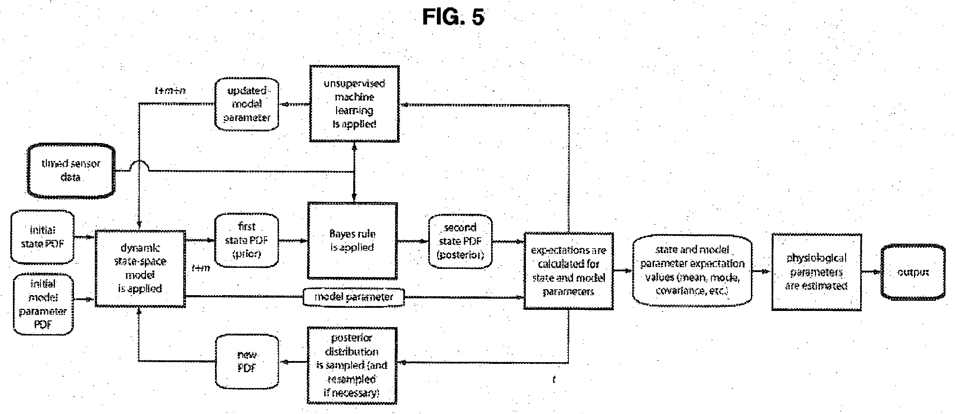

The state and parameter estimation steps may be coupled in an iterative dual-estimation mode as shown in **FIGs. 4 and 5.** This formulation for a state estimator operates on an adaptive DSSM. In the dual estimation process, states $\mathbf{x_k}$ and parameters **W** are estimated sequentially inside a loop. When used in a data processor for a pulse oximeter, the current state $\mathbf{x_k}$ from pulse oximeter sensor input $\mathbf{y_k}$. States $\mathbf{x_k}$, and parameters **W** are estimated sequentially inside a loop. Parameter estimates are passed from the previous iteration to state estimation for the current iteration. Several different implementations or variants of the SMC and SPKF methods exist, including the sigma-point, Gaussian-sum, and square-root forms. The particular choice may be influenced by the application.

The current estimate of the parameters $\mathbf{W_k}$ is used in the state estimator as a given (known) input, and likewise the current estimate of the state $\mathbf{x_k}$ is used in the parameter estimator. This results in a step-wise stochastic optimization within the combined state-parameter space.

The flow chart shown **FIG. 5** provides a summary of the steps involved in dual estimation process. Initial probability distributions for state and model parameters are provided to the DSSM to produce an initial probability distribution function (first PDF or prior PDF) representing the initial state. Data for a time $t_1$ from a sensor (new measurement) and the initial PDF are combined using a Bayesian statistical process to generate a second, posterior PDF that represents the state at the time of the measurement for the first sensor data. Expectation values for the second PDF are calculated, which may represent the most likely true value. Expectation values may also represent, for instance, the confidence interval or any statistical measure of uncertainty associated with the value. Based upon the expectation values, usually but not necessarily the values for state parameters having the highest probability of being correct, updated state parameters for time $t_1$ are combined with sensor data for time $t_1$ to update the model parameters for time $t_2$ in the DSSM by the process shown in **FIG. 4.** The expectation values are also fed into the DSSM as the state, in the form of a vector of state parameters (new PDF) as shown in **FIG. 4.** Once the state parameters and model parameters for the DSSM are updated to time $t_1$, the process is repeated with timed data for time $t_2$ to produce updated parameters for time $t_2$ and so forth. The time interval between time steps is usually constant such that time points may be described as *t, t+n, t+2n,* etc. If the time interval is not constant, then the time may be described using two or more time intervals as *t, t+n, t+n+m,* etc.

Joint Estimation Engine for Estimation of State and Model Parameters

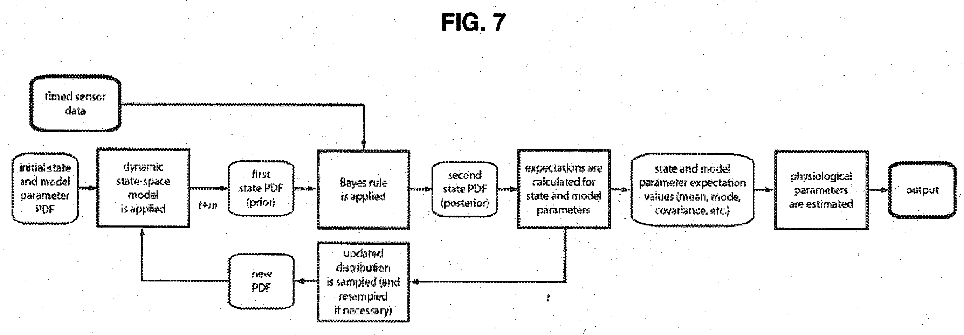

The state and parameter estimation steps may also be performed in a simultaneous joint-estimation mode as shown in **FIGs. 6 and 7.** The calculated variables for the state parameters and model parameters of the physiological model are concatenated into a single higher-dimensional joint state vector:

$$X = \begin{bmatrix} x_k^T & w_k^T \end{bmatrix}^T \tag{6}$$

where $x_k$-are the state parameters and $w_k$ the model parameters. The joint state space is used to produce simultaneous estimates of the states and parameters.

The flow chart shown **FIG. 7** provides a summary of the steps involved in joint estimation process. The process is similar to that shown for dual estimation in **FIG. 5,** with the exception that model and state parameters are not separated into two separate vectors, but are represented together in a single vector. The process is initiated by entering a vector representing initial state and model parameter value distributions into the DSSM and producing an initial first PDF. The first PDF is combined with sensor data (new measurement) for time $t_1$ in a Bayesian statistical process to generate a second, posterior PDF that represents the state and model parameters at time $t_1$. Expectation values for the second PDF are calculated, which may represent the most likely true value. Expectation values may also represent, for instance, the confidence interval or any statistical measure of uncertainty associated with the value. Based upon the expectation values, updated state and model parameters for time $t_1$ are entered into the DSSM by the process shown in **FIG. 6.** Once the state parameters and model parameters for the DSSM are updated to time $t_1$, the process is repeated with timed data for time $t_2$ to produce updated parameters for time $t_2$ and so forth.

Compared to dual estimation, both state and model parameters are concatenated into a single vector that is transformed by the dynamic state-space model. Hence, no machine learning step is necessary in order to update model parameters. Joint estimation may be performed using a sequential Monte Carlo method or sigma-point Kalman method. These may take the form of unscented, central difference, square-root unscented, and square-root central difference forms. The optimal method will depend on the particular application.

Sequential Monte Carlo Methods

SMC methods estimate the probability distributions of all the model unknowns by propagating a large number of samples called probability particles in accordance with the system models (typically nonlinear, non-Gaussian, non-stationary) and the rules of probability. Artifacts are equivalent to noise with short-lived probability distributions, also called non-stationary distributions. The number of simulated particles scales linearly with computational power, with $\geq 100$ particles being reasonable for real time processing with presently available processors. The system model describes pertinent physiology and the processor engine uses the system model as a "template" from which to calculate, using Bayesian statistics, posterior probability distribution functions (processed data). From this, the expectation values *(e.g.* the mean) and confidence intervals can be estimated **FIG. 7.** The combination of SMC with Bayesian Statistics to calculate posterior probability distribution functions is often referred to as a Particle Filter.

SMC process nonlinear and non-Gaussian problems by discretizing the posterior into weighted samples, or probability particles, and evolving them using Monte Carlo simulation. For discretization, Monte Carlo simulation uses weighted particles to map integrals to discrete sums:

$$p(\mathbf{x}_k | \mathbf{y}_{1:k}) \approx \hat{p}(\mathbf{x}_k | \mathbf{y}_{1:k}) = \sum_{i=1}^{N} \delta(\mathbf{x}_k - x_k^{(i)}) \tag{7}$$

where the random samples $\{x(i);\ i = 1, 2,...,N\}$, are drawn from $p(x_k|y_{1:k})$ and $\delta(.)$ is the Dirac delta function. Expectations of the form

$$E[\mathbf{g}(\mathbf{x}_k)] = \int \mathbf{g}(\mathbf{x}_k) p(\mathbf{x}_k | \mathbf{y}_{1:k}) d\mathbf{x}_k \tag{8}$$

can be approximated by the estimate:

$$E[\mathbf{g}(\mathbf{x}_k)] \approx \tilde{E}[\mathbf{g}(\mathbf{x}_k)] = \frac{1}{N} \sum_{i=1}^{N} \mathbf{g}(x_k^{(i)}) \tag{9}$$

if the distribution has finite support. As $N$ approaches infinity, the estimate converges to the true expectation.

The optimal Bayesian solution can be outlined by the following recursive algorithm. Suppose the required PDF $p(\mathbf{x}_{k-1}|\mathbf{y}_{1:k-1})$ at time $k$-1 is available. In the prediction stage, the prior PDF at time $k$ is obtained using the DSSM via the Chapman-Kolmogorov equation:

$$p(\mathbf{x}_k | \mathbf{y}_{1:k-1}) = \int p(\mathbf{x}_k | \mathbf{x}_{k-1}) p(\mathbf{x}_{k-1} | \mathbf{y}_{1:k-1}) d\mathbf{x}_{k-1} \tag{10}$$

The DSSM model describing the state evolution $p(\mathbf{x}_k|\mathbf{x}_{k-1})$ is defined by the system equation (1) and the known statistics of $v_{k-1}$. At time step $k$ a measurement $\mathbf{y}_k$ becomes available, and this may be used to update the prior (updated stage) via Bayes' rule:

$$p(\mathbf{x}_k | \mathbf{y}_{1:k}) = \frac{p(\mathbf{y}_k | \mathbf{x}_k) p(\mathbf{x}_k | \mathbf{y}_{1:k-1})}{p(\mathbf{y}_k | \mathbf{y}_{1:k-1})} \tag{11}$$

where the normalizing constant

$$p(\mathbf{y}_k | \mathbf{y}_{1:k-1}) = \int p(\mathbf{y}_k | \mathbf{x}_k) p(\mathbf{x}_k | \mathbf{y}_{1:k-1}) d\mathbf{x}_k \tag{12}$$

depends on the likelihood function $p(\mathbf{y}_k|\mathbf{x}_k)$ defined by the measurement model (equation 2) and the known statistics of $\mathbf{n}_k$.

It is not possible to sample directly from the posterior density function so importance sampling from a known proposal distribution $\pi(x_{0:k}|y_{1:k})$ is used. One may use sigma-point Kalman filters, for example, to generate the proposal.

The known distribution is introduced into Equation 5 to yield:

$$E[\mathbf{g}(\mathbf{x}_{0:k})] = \int \mathbf{g}(\mathbf{x}_{0:k}) \frac{w_k(\mathbf{x}_{0:k})}{p(\mathbf{y}_{1:k})} \pi(\mathbf{x}_{0:k} | \mathbf{y}_{1:k}) d\mathbf{x}_{0:k} \tag{13}$$

where the variables $w_k(x_{0:k})$ are unnormalized importance weights, which are written as $w_k(x_{0:k})= w_k$:

$$w_k(\mathbf{x}_{0:k}) = \frac{p(\mathbf{y}_{1:k}|\mathbf{x}_{0:k})p(\mathbf{x}_{0:k})}{\pi(\mathbf{x}_{0:k}|\mathbf{y}_{1:k})} \tag{14}$$

resulting in a weighted expectation:

$$E[\mathbf{g}(\mathbf{x}_{0:k})] \approx \tilde{E}[\mathbf{g}(\mathbf{x}_{0:k})] = \sum_{i=1}^{N} \tilde{w}_k^{(i)}\mathbf{g}(x_{0:k}^{(i)}) \tag{15}$$

where $\tilde{w}_k^{(i)}$ are normalized importance weights:

$$\tilde{w}_k^{(i)} = w_k^{(i)} \Big/ \sum_{j=1}^{N} w_k^{(j)} \tag{16}$$

Importance sampling is made sequential by reiterating the Markov 1st order assumption, resulting in the assumption that the current state is not dependent on future observations:

$$\pi(\mathbf{x}_{0:k}|\mathbf{y}_{1:k}) = \pi(\mathbf{x}_{0:k-1}|\mathbf{y}_{1:k-1})\pi(\mathbf{x}_k|\mathbf{x}_{0:k-1},\mathbf{y}_{1:k}) \tag{17}$$

and that observations are conditionally independent given the states:

$$p(\mathbf{x}_{0:k}) = p(\mathbf{x}_0)\prod_{j=1}^{k} p(\mathbf{x}_j|\mathbf{x}_{j-1}) \tag{18}$$

$$p(\mathbf{y}_{1:k}|\mathbf{x}_{0:k}) = \prod_{j=1}^{k} p(\mathbf{y}_j|\mathbf{x}_j) \tag{19}$$

A recursive estimate for the importance weights is:

$$w_k = w_{k-1}\frac{p(\mathbf{y}_k|\mathbf{x}_k)p(\mathbf{x}_k|\mathbf{x}_{k-1})}{\pi(\mathbf{x}_k|\mathbf{x}_{0:k-1},\mathbf{y}_{1:k})} \tag{20}$$

which is called Sequential Importance Sampling (SIS). SIS suffers from degeneracy so that, over a few iterations, all but one of the importance weights will be zero, effectively removing a large number of samples. To remedy this, samples with low importance weights may be eliminated while high importance samples may be multiplied. One way to accomplish this is Sampling-Importance Resampling (SIR), which involves mapping the Dirac random measure

$$\{x_k^{(i)}, \tilde{w}_k^{(i)}; i = 1,\ldots,N\} \tag{21}$$

into a measure with equal weights, $1/N$:

$$\{x_k^{(i)}, \frac{1}{N}; i = 1,\ldots,N\} \tag{22}$$

A pseudo-code for a generic SMC (also called bootstrap filter or condensation algorithm) can be written as:

1. Importance sampling step. For i = 1,...,$N$, do:

i) sample

$$x_k^{(i)} \sim p(x_k | x_{k-1}^{(i)})$$

ii) evaluate

$$w_k^{(i)} \sim w_{k-1}^{(i)} p(y_k | x_k^{(i)})$$

iii) normalize

$$\tilde{w}_k^{(i)} = w_k^{(i)} / \Sigma_{j=1}^{N} w_k^{(j)}$$

2. Importance resampling step

i) eliminate or multiply samples $x_k^{(i)}$ according to weights $\tilde{w}_k^{(i)}$ obtain N random samples approximately distributed according to $p(x_k|y_{k-1})$.

ii) For $i = 1,..., N$, set

$$w_k^{(i)} = \tilde{w}_k^{(i)} = N^{-1}$$

3. Output

i) any expectation, for instance:

$$\hat{x}_k = E[x_k | y_{1:k}] \approx \frac{1}{N} \sum_{i=1}^{N} x_k^{(i)}$$

SPKF may be used to approximate probability distributions. Assuming that x has a mean $\overline{X}$:ovariance $\mathbf{P}_x$, and dimension $L$, a set of 2L+1 weighted sigma-points, $S_i = \{w_i, X_k\}$, is chosen according to:

$$\mathbf{X}_0 = \bar{\mathbf{x}}$$
$$w_0^{(m)} = \frac{h^2 - L}{h^2}$$

$$\mathbf{X}_i = \bar{\mathbf{x}} + (h\sqrt{\mathbf{P}_x})_i \quad i = 1,\ldots,L \qquad w_i^{(m)} = \frac{1}{2h^2} \qquad i = 1,\ldots,2L$$

$$\mathbf{X}_i = \bar{\mathbf{x}} - (h\sqrt{\mathbf{P}_x})_i \quad i = L+1,\ldots,2L \qquad w_i^{(c)} = \frac{1}{4h^2} \qquad i = 1,\ldots,2L$$

$$w_i^{(s)} = \frac{h^2 - 1}{4h^4} \qquad i = 1,\ldots,2L \qquad (23)$$

where $h$ is a scaling parameter. Each sigma-point is propagated through the DSSM to yield the posterior sigma-point set, $\mathbf{Y}_i$:

$$\mathbf{Y}_i = \mathbf{h}(\mathbf{f}(\mathbf{X}_i)), \ i = 0,\ldots,2L \qquad (24)$$

From this, the posterior statistics are calculated using a procedure resembling the linear Kalman filter. For instance, for the unscented Kalman filter case, a SPKF variant, the time-update equations are:

$$\mathbf{X}_{k|k-1}^x = \mathbf{f}(\mathbf{X}_{k-1}^x, \mathbf{X}_{k-1}^v, \mathbf{u}_{k-1}) \qquad (25)$$

$$\hat{\mathbf{x}}_k^- = \sum_{i=0}^{2L} w_i^{(m)} \mathbf{X}_{i,k|k-1}^x \qquad (26)$$

$$\mathbf{P}_{x_k}^- = \sum_{i=0}^{2L} w_i^{(c)} (\mathbf{X}_{i,k|k-1}^x - \hat{\mathbf{x}}_k^-)(\mathbf{X}_{i,k|k-1}^x - \hat{\mathbf{x}}_k^-)^T$$

and the measurement-update equations are:

$$\mathbf{Y}_{k|k-1} = \mathbf{h}(\mathbf{X}_{k|k-1}^x - \mathbf{X}_{k-1}^n) \qquad (28)$$

$$\hat{\mathbf{y}}_k^- = \sum_{i=0}^{2L} w_i^{(m)} \mathbf{Y}_{i,k|k-1} \qquad (29)$$

$$\mathbf{P}_{\hat{y}_k} = \sum_{i=0}^{2L} w_i^{(c)} (\mathbf{Y}_{i,k|k-1} - \hat{\mathbf{y}}_k^-)(\mathbf{Y}_{i,k|k-1} - \hat{\mathbf{y}}_k^-)^T \qquad (30)$$

$$\mathbf{P}_{x_k y_k} = \sum_{i=0}^{2L} w_i^{(c)} (\mathbf{X}_{i,k|k-1}^x - \hat{\mathbf{x}}_k^-)(\mathbf{Y}_{i,k|k-1} - \hat{\mathbf{y}}_k^-)^T \qquad (31)$$

$$\mathbf{K}_k = \mathbf{P}_{x_k y_k} \mathbf{P}_{\hat{y}_k}^{-1} \qquad (32)$$

$$\hat{\mathbf{x}}_k = \hat{\mathbf{x}}_k^- + \mathbf{K}_k (\mathbf{y}_k - \hat{\mathbf{y}}_k^-) \qquad (33)$$

$$\mathbf{P}_{x_k} = \mathbf{P}_{x_k}^- - \mathbf{K}_k \mathbf{P}_{\hat{y}_k} \mathbf{K}_k^T \qquad (34)$$

where $x$, $v$ and n superscripts denote the state, process noise and measurement noise dimensions, respectively.

The mathematical structure for sequential Monte Carlo and SPKF represent two examples of a family of probabilistic inference methods exploiting Monte Carlo simulation and the sigma point transform, respectively, in conjunction with a Bayesian statistical process.

SPKF are generally inferior to SMC but are computationally cheaper. Like SMC, SPKF evolve the state using the full nonlinear DSSM, but represent probability distributions using a sigma-point set. This is a deterministic step that replaces the stochastic Monte Carlo step in the SMC. As a result, SPKF lose accuracy when posterior distributions depart heavily from the Gaussian form, such as with bimodal or heavily-tailed distributions, or with strong nonstationary distributions such as those caused by motion artifacts in pulse oximeters. For these cases SMC are more suitable.

SPKF yields higher-order accuracy than the extended Kalman filter (EKF) and its related variants with equal algorithm complexity, $O(L^2)$. SPKF returns 2nd order accuracy for nonlinear and non-Gaussian problems, and 3rd order for Gaussian problems. EKF has only 1st order accuracy for nonlinear problems. Both EKF and SPKF approximate state distributions with Gaussian random variables (GRV). However, the EKF propagates the GRV using a single measure (usually the mean) and the 1st order Taylor expansion of the nonlinear system. The SPKF, on the other hand, decomposes the GRV into distribution moments (sigma points) and propagates those using the unmodified nonlinear system. SPKF implementation is simpler than EKF since it is derivativeless. That is, it uses the unmodified DSSM form, and therefore does not require lengthy Jacobian derivations.

The data processing method is also capable of prediction because the method can operate faster than real time measurements. At any given time during data processing, the measurement PDF, obtained either from SPKF or SMC, embodies all available statistical information up to that point in time. It is therefore possible to march the system model forwards in time, for instance, using the same sequential Monte Carlo method, to obtain deterministic or stochastic simulations of future signal trajectories. In this way, the future health status (physiological state) of a patient can be predicted with attached probabilities indicating the confidence of each prediction.

Noise Adaptation

The data processing method may benefit from a noise adaptation method if timed sensor data contains noise and/or artifact that changes its spectral qualities over time. That is, has a non-stationary probability distribution function. Here, a known algorithm such as the Robins-Monro or Annealing methods may be added to the data processing method in order to adapt the probability distribution functions of noise terms (stochastic terms) in the DSSM according to changing noise and artifact present in sensor data.

Output

In general, the output may include estimates of the true measured signals (i.e. processed data), and estimates of values for one or more physiological parameters measured by one or more sensors from which data was received, and estimates of values for one or more physiological parameters not measured by the sensors from which data was received (data extraction). A state parameter estimate is the processed data from the physiological sensor. Both noise and artifacts can be attenuated or rejected even though they may have very distinct probability distribution functions and may mimic the real signal. A model parameter estimate may be also used to produce a physiological parameter. For example, an estimate of total blood volume may be used to diagnose hemorrhage or hypovolemia; an estimate of tissue oxygen saturation may indicate poor tissue perfusion and/or hypoxia; estimates of glucose uptake in several tissues may differentiate between diabetes mellitus types and severities; and estimates of carotid artery radius may be indicative of carotid artery stenosis.

EXAMPLES:

Pulse Oximeter with Probabilistic Data Processing

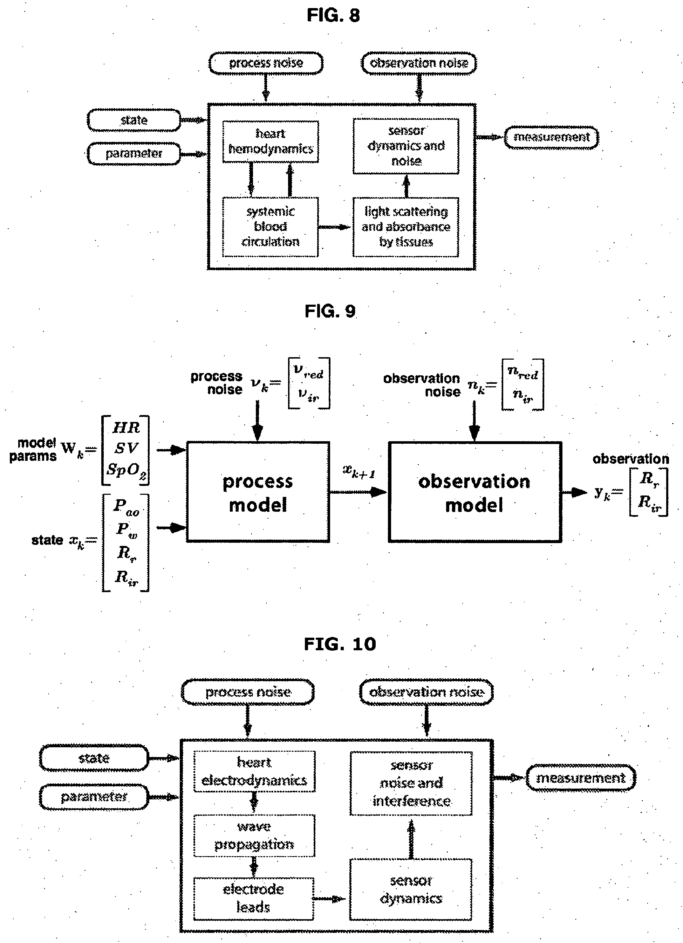

**FIG. 8** shows the components of a DSSM suitable for processing data from a pulse oximeter model, including components required to describe processes occurring in a subject. **FIG. 9** illustrates the DSSM broken down into process and observation models, and including all input and output variables. Heart rate ($HR$), stroke volume ($SV$) and whole-blood oxygen saturation ($SpO_2$) are estimated from input noisy red and infrared intensity ratios ($R$). Radial $(Pw)$ and aortic (Pao) pressures are also available as state estimates.

In this example, the DSSM comprises the following function to represent cardiac output:

$$Q_{CO}(t) = \bar{Q}_{CO} \sum_{k=1}^{6} a_k \exp\left[\frac{-(t-b_k)^2}{c_k^2}\right] \tag{31}$$

wherein cardiac output Qco(t), is expressed as a function of heart rate ($HR$) and stroke volume ($SV$) and where $Q_{CO} = (HR \times SV)/60$. The cardiac output function pumps blood into a Windkessel 3-element model of the vascular system including two state variables: aortic pressure, Pao, and radial (Windkessel) pressure, $Pw$:

$$P_{w,k+1} = \frac{1}{C_p R_p}\left((R_p + Z_o)Q_{CO} - P_{w,k}\right) \cdot \delta t + P_{w,k} \tag{32}$$

$$P_{ao,k+1} = P_{w,k+1} + Z_o Q_{CO} \tag{33}$$

$R$p and Zo are the peripheral resistance and characteristic aortic impedance, respectively. The sum of these two terms is the total peripheral resistance due to viscous (Poiseuille-like) dissipation:

$$Z_o = \sqrt{\rho/AC_l} \tag{34}$$

where $\rho$ is blood density. The elastic component due to vessel compliance is a nonlinear function including thoracic aortic cross-sectional area, $A$:

$$A(P_{ao}) = A_{max}\left[\frac{1}{2} + \frac{1}{\pi}\arctan\left(\frac{P_{ao} - P_0}{P_1}\right)\right] \tag{35}$$

where $A$max, $P_0$ and $P_1$ are fitting constants correlated with age and gender:

$$A_{max} = (5.62 - 1.5(gender)) \cdot cm^2 \tag{36}$$

$$P_s = (76 - 4(gender) - 0.89(age)) \cdot mmHg \tag{37}$$

$$P_i = (57 - 0.44(age)) \cdot mmHg \tag{38}$$

The time-varying Windkessel compliance, $Cw$, and the aortic compliance per unit length, Cl, are:

$$C_w = lC_i = l\frac{dA}{dP_w} = l\frac{A_{max}/(\pi P_i)}{1 + \left(\frac{P_w - P_0}{P_i}\right)^2} \tag{39}$$

where $l$ is the aortic effective length. The peripheral resistance is defined as the ratio of average pressure to average flow. A set-point pressure, $P$set, and the instantaneous flow:

$$R_p = \frac{P_{set}}{(HR \cdot SV)/60} \tag{40}$$

are used to provide compensation autonomic nervous system responses. The value for $P$set is adjusted manually to obtain 120 over 75 mmHg for a healthy individual at rest. The compliance of blood vessels changes the interactions between light and tissues with pulse. This is accounted for using a homogenous
photon diffusion theory for a reflectance or transmittance pulse oximeter configuration. For the reflectance case:

$$R = \frac{I_{ac}}{I_{dc}} = \frac{\Delta I}{I} = \frac{3}{2}\Sigma_s K(\alpha, d, r)\Sigma_s^{art}\Delta V_a \tag{41}$$

for each wavelength. In this example, the red and infrared bands are centered at ~ 660 nm and ~ 880 nm. I denotes the detected intensities: total reflected (no subscript), and the pulsating (ac) and background (dc) components. Va is the arterial blood volume, which changes as the cross-sectional area of illuminated blood vessels, $\Delta A_w$, changes as:

$$\Delta V_a = r \cdot \Delta A_w \tag{42}$$

where r is the source-detector distance. The tissue scattering coefficient, $\Sigma$s', is assumed constant but the arterial absorption coefficient, $\Sigma a^{art}$, depends on blood oxygen saturation, $SpO_2$:

$$\Sigma_a^{art} = \frac{H}{v_i}\left[SpO_2 \cdot \sigma_a^{100\%} + (1 - SpO_2) \cdot \sigma_a^{0\%}\right] \tag{43}$$

which is the Beer-Lambert absorption coefficient, with hematocrit, H, and red blood cell volume, $v_i$. The optical absorption cross sections for red blood cells containing totally oxygenated ($HbO_2$) and totally deoxygenated (Hb) hemoglobin are $\sigma_a^{100\%}$ and $\sigma_a^{0\%}$, respectively.

The function K($\alpha$,d,r) contains, along with the scattering coefficient, the wavelength, sensor geometry and oxygen saturation dependencies that alter the effective optical pathlengths:

$$K(\alpha, d, r) \approx \frac{-r^2}{1 + \alpha r} \tag{44}$$

The attenuation coefficient $\alpha$ is:

$$\alpha = \sqrt{3\Sigma_a(\Sigma_a + \Sigma_s)} \tag{45}$$

where $\Sigma_a$ and $\Sigma_s$ are whole-tissue absorption and scattering coefficients, respectively, which are calculated from Mie Theory.

Red and infrared K values as a function of $SpO_2$ may be represented by two linear. fits:

$$\overline{K}_r \approx -4.03 \cdot SpO_2 - 1.17 \tag{46}$$

$$\overline{K}_{ir} \approx 0.102 \cdot SpO_2 - 0.753 \tag{47}$$

in mm$^2$. The overbar denotes the linear fit of the original function. The pulsatile behavior of $\Delta Aw$, which couples optical detection with the cardiovascular system model, is:

$$\Delta A_w = \frac{A_{w,max}}{\pi} \frac{P_{w,1}}{P_{w,1}^2 + (P_{w,k+1} - P_{w,0})^2} \Delta P_w \tag{48}$$

with $P_{w,0}=(1/3)P_0$ and $P_{w,1}=(1/3)P_1$ to account for the poorer compliance of arterioles and capillaries relative to the thoracic aorta. Third and fourth state variables, the red and infrared reflected intensity ratios, R=Iac/Idc, are:

$$R_{r,k+1} = c\Sigma_{a,r}' \overline{K}_r \Sigma_{a,r}^{eff} \Delta A_w + R_{r,k} + v_r \tag{49}$$

$$R_{ir,k+1} = c\Sigma_{a,ir}' \overline{K}_{ir} \Sigma_{a,ir}^{eff} \Delta A_w + R_{ir,k} + v_{ir} \tag{50}$$

Here, $v$ are Gaussian-distributed process noises intended to capture the baseline wander of the two channels. The constant c subsumes all factors common to both wavelengths and is treated as a calibration constant. The observation model adds Gaussian-distributed noises, $n$, to $R_r$ and $R_{ir}$:

$$\begin{Bmatrix} y_{r,k} \\ y_{ir,k} \end{Bmatrix} = \begin{Bmatrix} R_{r,k} \\ R_{ir,k} \end{Bmatrix} + \begin{Bmatrix} n_{r,k} \\ n_{ir,k} \end{Bmatrix} \tag{51}$$

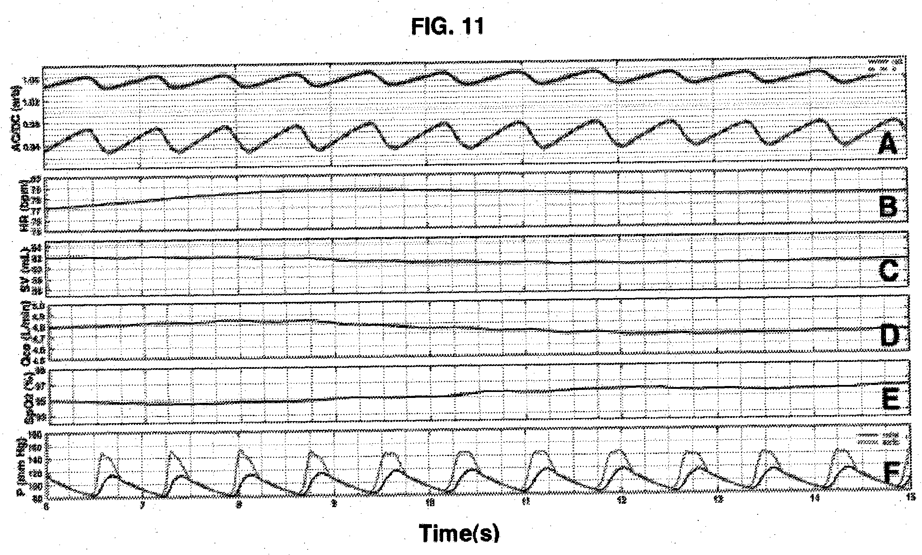

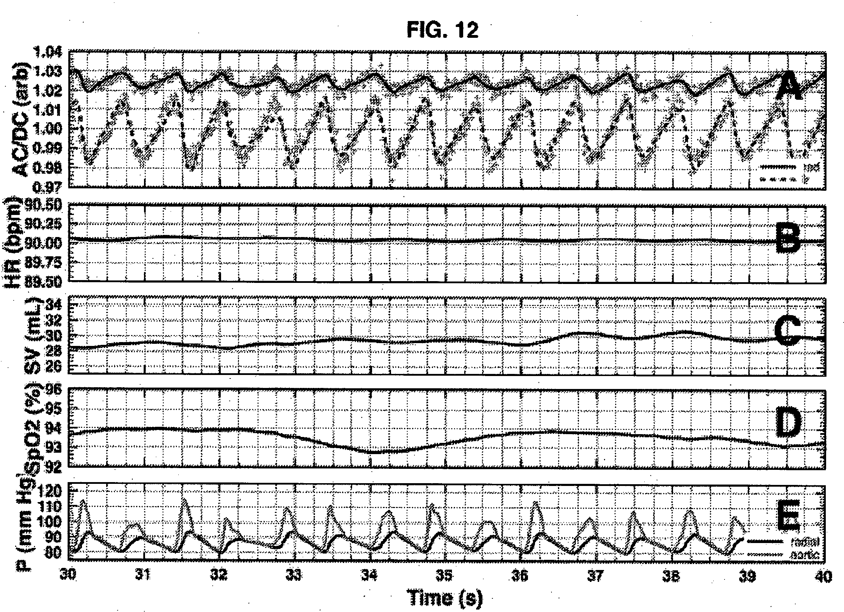

A calibration constant c was used to match the variance of the real Iac/Idc signal with the variance of the DSSM-generated signal for each wavelength. After calibration, the age and gender of the patient was entered. Estimates for the means and covariances of both state and parameter PDFs were entered. **FIG. 11** plots estimates for a 15 s stretch of data. Photoplethysmographic waveforms (A) were used to extract heart rate **(B),** left-ventricular stroke volume **(C),** cardiac output **(D),** blood oxygen saturation **(E),** and aortic and systemic (radial) pressure waveforms **(F).** Results of processing pulse oximetry at low blood perfusion are shown in **FIG. 12.** Low signal-to-noise photoplethysmographic waveforms **(A)** were used to extract heart rate **(B),** left-ventricular stroke volume **(C),** blood oxygen saturation **(D),** and aortic and systemic (radial) pressure waveforms **(E).**

Electrocardiograph with Probabilistic Data Processing

**FIG. 10** is a schematic of a dynamic state-space model suitable for processing electrocardiograph data, including components required to describe the processes occurring in a subject. The combination of SPKF or SMC in state, joint or dual estimation modes can be used to filter electrocardiography (ECG) data. Any physiology model adequately describing the ECG signal can be used, as well as any model of noise and artifact sources interfering or contaminating the signal. One non-limiting example of such a model is the ECG signal generator proposed by McSharry (IEEE Transactions on Biomedical Engineering, 2003. 50(3):289-294). Briefly, this model uses a sum of Gaussians with amplitude, center and standard deviation, respectively, for each wave (P, Q, R, S, T-, T+15) in an ECG. The observation model comprises the state plus additive Gaussian noise, but more realistic pink noise or any other noise distributions can be used.

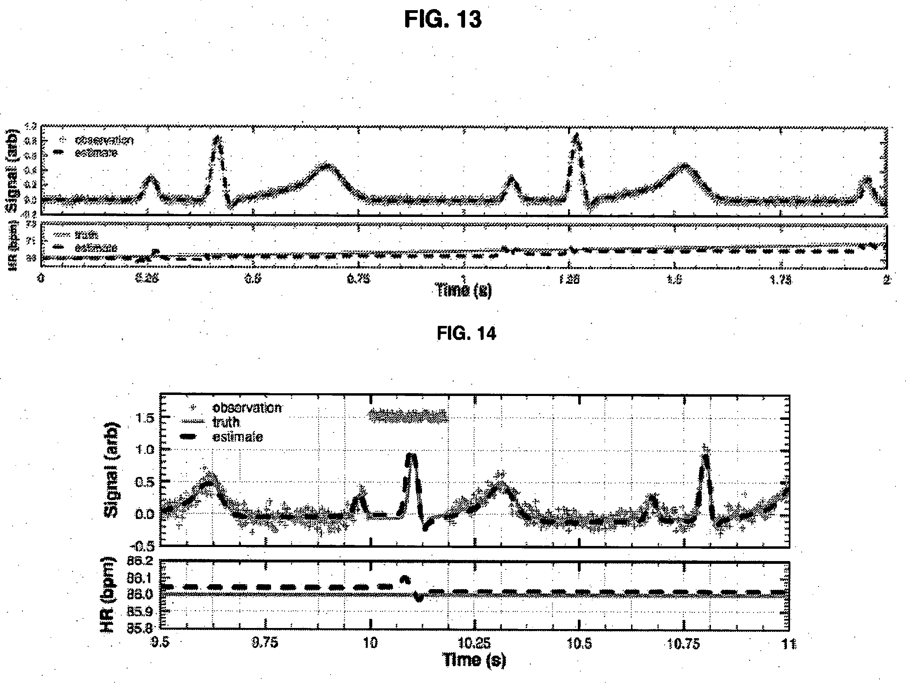

**FIG. 13** shows the results of processing a noisy non-stationary ECG signal. Heart rate oscillations representative of normal respiratory sinus-arrhythmia are present in the ECG. The processor accomplishes accurate, simultaneous estimation of the true ECG signal and heart rate that follows closely the true values. The performance of the processor in a noise and artifact-corrupted signal is shown in **FIG. 14.** A clean ECG signal representing one heart beat (truth) was contaminated with additive noise and an artifact in the form of a plateau at R and S peaks (beginning at time = 10 s). Estimates by the processor remain close to the true signal despite the noise and artifact.

Specific DSSMs and input and output parameters are provided for the purpose of describing the present method, but the present invention is not intended to be limited to the DSSMs, sensors, biological monitoring devices, inputs, outputs used to describe the method. The scope of the invention is limited only by the appended claims.

**Claims**

1. A computer-implemented method for processing pulse oximeter data using a dynamic state space model (DSSM) to extract an estimated value for at least one physiological parameter not measured by said pulse oximeter, said method comprising:

   a) entering system and model parameters for a time t into a dynamic state space model to produce a first probability distribution function (PDF) vector comprising state and model parameters for time t+n;
   b) using the first PDF vector and timed data obtained for time t+n from the sensor in a Bayesian statistical process to produce a second PDF vector for state and model parameters for time t+n;
   c) calculating probabilistic expectation values for the state and model parameters for time t+n from the second probability distribution function; and
   d) determining an estimated value for the at least one physiological parameter for time t+n from probabilistic expectation values for the state and/or model parameters for time t+nwherein:

   the state and model parameters for a time t entered into the dynamic state-space model in step a) are in the form of a probability distribution function produced from a sampling of expectation values calculated in step c) for an immediately preceding time t-n'; and n and n' are time intervals that may be the same of different **characterized in that**:

   the DSSM mathematically represents physiological processes that produce physiological parameters measured by said pulse oximeter and said at least one physiological parameter not measured by said pulse oximeter to produce a time dependent state representing a time dependent physiological state of the subject,
   the DSSM comprises at least one model parameter and/or state parameter representing at least one of total blood volume (TBV), stroke volume (SV), vasomotor tone (VT), and autonomous nervous system (ANS) tone and
   the at least one physiological parameter not measured by said pulse oximeter is one of total blood volume (TBV), stroke volume (SV), vasomotor tone (VT), and autonomous nervous system (ANS) tone.

2. The method of claim 1, **characterized in that** said estimated value determined in step d) is equal to the value of a model parameter or a state parameter of said DSSM, or is calculated from the value of a model parameter and/or a state parameter of said DSSM.

3. The method of claim 1 or 2, **characterized in that** the DSSM is integrated in a joint estimation processing engine.

4. The method of any preceding claim, **characterized in that** the first PDF is produced using a Sequential Monte Carlo or Sigma Point Kalman Filter method.

5. The method of claim 5, **characterized in that** the Sigma Point Kalman Filter method is an unscented Kalman Filter, a central difference Kalman Filter, a square-root unscented Kalman Filter, a square-root central difference Kalman Filter, or a combination thereof; and the Sequential Monte Carlo method is an unscented Monte Carlo method, a central difference Monte Carlo method, a square-root unscented Monte Carlo method, a square-root central difference Monte Carlo method, Gaussian Sum Monte Carlo method, Bayes Monte Carlo method, a Gaussian Mixture Sigma Point Monte Carlo method, or a combination thereof.

6. The method of any preceding claim, **characterized in that** the at least one physiological parameter not measured by said pulse oximeter is total blood volume (TBV) and/or stroke volume (SV).

7. A computer program that, when loaded in a computer, is operational to implement steps a) - d) of claim 1.

8. A computer loaded with the program of claim 7.

9. A system for estimating stroke volume and/or total blood volume, said system comprising a pulse oximeter and a computer configured to perform the method of claim 6.


**Patentansprüche**

1. Computerimplementiertes Verfahren zur Verarbeitung von Pulsoximeterdaten unter Verwendung eines dynamischen Zustandsraummodells (Dynamic State Space Model, DSSM) zum Extrahieren eines geschätzten Werts für mindestens einen nicht durch das Pulsoximeter gemessenen physiologischen Parameter, wobei das Verfahren umfasst:

   a) Eingeben von System-und Modellparametern für eine Zeit t in ein dynamisches Zustandsraummodell, um einen ersten Wahrscheinlichkeitsverteilungsfunktions (PDF)-Vektor zu erzeugen, umfassend Zustands- und Modellparameter für die Zeit t+n;
   b) Verwenden des ersten PDF-Vektors und der für die Zeit t+n vom Sensor in einem statistischen bayesschen Prozess erhaltenen zeitlich festgelegten Daten, um einen zweiten PDF-Vektor für Zustands- und Modellparameter für die Zeit t+n zu erzeugen;
   c) Berechnen probabilistischer Erwartungswerte für die Zustands- und Modellparameter für die Zeit t+n aus der zweiten Wahrscheinlichkeitsverteilungsfunktion; und
   d) Bestimmen eines geschätzten Werts für den mindestens einen physiologischen Parameter für t+n aus den probabilistischen Erwartungswerten für die Zustands- und/oder Modellparameter für die Zeit t+n, wobei:

   die in Schritt a) für eine Zeit t in das dynamische Zustandsraummodell eingegebenen Zustands- und Modellparameter in der Form einer aus einer in Schritt c) für eine unmittelbar vorhergehende Zeit t-n' erzeugten Probennahme von Erwartungswerten berechneten Wahrscheinlichkeitsverteilungsfunktion sind; und n und n' sind Zeitintervalle, die gleich oder verschieden sein können
   **dadurch gekennzeichnet, dass:**

   das DSSM physiologische Prozesse, die die durch das Pulsoximeter gemessenen physiologischen Parameter erzeugen, und den mindestens einen nicht durch das Pulsoximeter gemessenen physiologischen Parameter mathematisch repräsentiert, um einen, einen zeitabhängigen physiologischen Zustand des Patienten repräsentierenden, zeitabhängigen Zustand zu erzeugen,
   das DSSM mindestens einen Modellparameter und/oder Zustandsparameter umfasst, repräsentierend mindestens eins von Gesamtblutvolumen (TBV),
   Herzschlagvolumen (SV), Vasomotortonus (VT) und autonomes Nervensystem (ANS)tonus und
   der mindestens eine nicht durch das Pulsoximeter gemessene physiologische Parameter eins von Gesamtblutvolumen (TBV), Herzschlagvolumen (SV), Vasomotortonus (VT) und autonomes Nervensystem (ANS)tonus ist.

**2.** Verfahren nach Anspruch 1, **dadurch gekennzeichnet, dass** der in Schritt d) bestimmte geschätzte Wert gleich dem Wert eines Modellparameters oder eines Zustandsparameters des DSSM ist, oder aus dem Wert eines Modellparameters und/oder eines Zustandsparameters des DSSM berechnet ist.

**3.** Verfahren nach Anspruch 1 oder 2, **dadurch gekennzeichnet, dass** das DSSM in einer gemeinsamen Schätzungsverarbeitungsmaschine integriert ist.

**4.** Verfahren nach einem vorstehenden Anspruch, **dadurch gekennzeichnet, dass** die erste PDF unter Verwendung einer sequenziellen Monte-Carlo- oder Sigma Point Kalman-Filter-Methode erzeugt wird.

**5.** Verfahren nach Anspruch 5, **dadurch gekennzeichnet, dass** die Sigma Point Kalman-Filter-Methode ein Unscented-Kalman-Filter, ein Central-Difference-Kalman-Filter, ein Square-Root-Unscented-Kalman-Filter, ein Square-Root-Central-Difference-Kalman-Filter oder eine Kombination davon ist; und die sequenzielle Monte-Carlo-Methode eine Unscented-Monte-Carlo-Methode, eine Central-Difference-Monte-Carlo-Methode, eine Square-Root-Unscented-Monte-Carlo-Methode, eine Square-Root-Central-Difference-Monte-Carlo-Methode, Gaußsche-Summe-Monte-Carlo-Methode, Bayes-Monte-Carlo-Methode, eine Gaußsche-Mischung-Sigma Point-Monte-Carlo-Methode oder eine Kombination davon ist.

**6.** Verfahren nach einem vorstehenden Anspruch, **dadurch gekennzeichnet, dass** der mindestens eine nicht durch das Pulsoximeter gemessene physiologische Parameter das Gesamtblutvolumen (TBV) und/oder Herzschlagvolumen (SV) ist.

**7.** Computerprogramm, das, wenn in einen Computer geladen, betriebsfähig ist, Schritte a) - d) von Anspruch 1 zu implementieren.

**8.** Computer, mit dem geladenen Programm von Anspruch 7.

**9.** System zum Schätzen von Herzschlagvolumen und/oder Gesamtblutvolumen, wobei das System ein Pulsoximeter und einen zum Durchführen des Verfahrens von Anspruch 6 konfigurierten Computer umfasst.

**Revendications**

**1.** Procédé mis en oeuvre par ordinateur pour traiter des données de sphygmo-oxymètre utilisant un modèle état-espace dynamique (DSSM) pour extraire une valeur estimée pour au moins un paramètre physiologique non mesuré par ledit sphygmo-oxymètre, ledit procédé comprenant :

a) la saisie de paramètres de système et de modèle pour un temps t dans un modèle état-espace dynamique pour produire un premier vecteur de fonction de distribution de probabilité (PDF) comprenant des paramètres d'état et de modèle pour le temps t+n ;
b) l'utilisation d'un premier vecteur PDF et des données datées obtenus pour le temps t+n provenant du capteur dans un procédé statistique bayésien pour générer un second vecteur PDF pour les paramètres d'état et de modèle pour le temps t+n ;
c) le calcul des valeurs d'anticipation probabilistiques pour les paramètres d'état et de modèle pour le temps t+n à partir de la seconde fonction de distribution de la probabilité ; et
d) la détermination d'une valeur estimée pour ledit au moins un paramètre physiologique pour le temps t+n à partir des valeurs d'anticipation probabilistiques pour les paramètres d'état et/ou de modèle pour le temps t+n dans lequel :

les paramètres d'état et de modèle pour un temps t saisis dans le modèle état-espace dynamique à l'étape a) sont sous la forme d'une fonction de distribution de la probabilité produite à partir d'un échantillonnage de valeurs d'anticipation calculées à l'étape c) pour un temps immédiatement précédent t-n' ; et n et n' représentent des intervalles de temps qui peuvent être identiques ou différents
**caractérisé en ce que** :

le DSSM représente mathématiquement les processus physiologiques qui produisent les paramètres physiologiques mesurés par ledit sphygmo-oxymètre et ledit au moins un paramètre physiologique non mesuré par ledit sphygmo-oxymètre pour produire un état dépendant du temps représentant un état physiologique dépendant du temps du sujet,

le DSSM comprend au moins un paramètre de modèle et/ou un paramètre d'état représentant au moins un paramètre parmi un volume de sang total (VST), d'un débit systolique (DS), une tension vasomotrice (TV) et une tension du système nerveux autonome (TSNA) et

ledit au moins un paramètre physiologique non mesuré par ledit sphygmo-oxymètre est un paramètre parmi d'un volume de sang total (VST), un débit systolique (DS), une tension vasomotrice (TV) et une tension du système nerveux autonome (TSNA).

2. Procédé selon la revendication 1, **caractérisé en ce que** ladite valeur estimée déterminée à l'étape d) est égale à la valeur d'un paramètre de modèle ou d'un paramètre d'état dudit DSSM, ou est calculée à partir d'une valeur d'un paramètre de modèle ou d'un paramètre d'état dudit DSSM.

3. Procédé selon la revendication 1 ou 2, **caractérisé en ce que** le DSSM est intégré dans un moteur de traitement d'estimation commune.

4. Procédé selon l'une quelconque revendication précédente, **caractérisé en ce que** le premier PDF est produit par un procédé de Monte-Carlo séquentiel ou à filtre de Kalman déterministe (Sigma-Point).

5. Procédé selon la revendication 5, **caractérisé en ce que** le procédé de filtre de Kalman déterministe (Sigma-Point) est un filtre de Kalman inodore, un filtre de Kalman à différence centrale, un filtre de Kalman à racine carrée, un filtre de Kalman à racine carrée et différence centrale, ou une combinaison de ceux-ci ; et le procédé de Monte-Carlo séquentiel est un procédé de Monte-Carlo inodore, un procédé de Monte-Carlo à différence centrale, un procédé de Monte-Carlo à racine carrée inodore, un procédé de Monte-Carlo à racine carrée et différence centrale, un procédé de Monte-Carlo à somme gaussienne, un procédé de Monte-Carlo de Bayes, un procédé de Monte-Carlo déterministe gaussien mixte, ou une combinaison de ceux-ci.

6. Procédé selon l'une quelconque revendication précédente, **caractérisé en ce que** ledit au moins un paramètre physiologique non mesuré par ledit sphygmo-oxymètre est le volume de sang total (VST) et/ou le débit systolique (DS).

7. Programme informatique qui, lorsqu'il est chargé dans un ordinateur, est opérationnel pour mettre en oeuvre les étapes a) à d) de la revendication 1.

8. Ordinateur chargé du programme de la revendication 7.

9. Système permettant d'estimer le débit systolique et/ou le volume de sang total, ledit système comprenant un sphygmo-oxymètre et un ordinateur configuré pour exécuter le procédé de la revendication 6.

## Fig. 1

```
┌──────────┐      ┌──────────┐      ┌──────────┐      ┌──────────┐
│Biomedical│ ──▶  │ Analog   │ ──▶  │  Signal  │ ──▶  │Analog to │
│ Sensor   │      │ Raw      │      │Conditioner│     │ Digital  │
│          │      │ Signal   │      │          │      │Converter │
└──────────┘      └──────────┘      └──────────┘      └──────────┘
                                                            │
                                                            ▼
┌──────────┐      ┌──────────┐      ┌──────────┐      ┌──────────┐
│ Output   │ ◀──  │ Clean    │ ◀──  │  Data    │ ◀──  │ Digital  │
│ Display  │      │ Signal and│     │Processor │      │ Raw      │
│          │      │ Diagnosis│      │          │      │ Signal   │
└──────────┘      └──────────┘      └──────────┘      └──────────┘
```

## Fig. 2

```
            ┌──────────────┐              ┌──────────────────┐
            │ process noise│              │observation noise │
            └──────────────┘              └──────────────────┘
                    │                              │
                    ▼                              ▼
  ┌───────┐   ┌──────────────┐        ┌──────────────────┐
  │ state │──▶│   PROCESS    │        │   OBSERVATION    │     ┌─────────────┐
  └───────┘   │    MODEL     │ ─────▶ │      MODEL       │ ──▶ │ measurement │
 ┌──────────┐ │ (time update)│        │                  │     └─────────────┘
 │parameter │▶│              │        │                  │
 └──────────┘ └──────────────┘        └──────────────────┘
```

## Fig. 3

```
               v_k                          n_k
                │                            │
                ▼                            ▼
  W_k ──▶ ┌──────────┐             ┌──────────┐
          │          │   x_{k+1}   │          │
          │    F     │ ──────────▶ │    H     │ ──▶ y_k
  x_k ──▶ │          │             │          │
          └──────────┘             └──────────┘
```

**Fig. 4**

**Fig. 6**

FIG. 5

EP 2 512 325 B1

**FIG. 7**

## FIG. 8

## FIG. 9

## FIG. 10

FIG. 11

EP 2 512 325 B1

Time(s)

FIG. 12

**FIG. 13**

**FIG. 14**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20090069647 A

**Non-patent literature cited in the description**

- **MCSHARRY.** *IEEE Transactions on Biomedical Engineering,* 2003, vol. 50 (3), 289-294